(19) Europäisches Patentamt
European Patent Office
Office européen des brevets

(11) EP 2 150 234 B1

(12) EUROPEAN PATENT SPECIFICATION

(45) Date of publication and mention of the grant of the patent:
**17.04.2013 Bulletin 2013/16**

(21) Application number: **08873888.5**

(22) Date of filing: **19.03.2008**

(51) Int Cl.:
***A61K 9/08*** (2006.01) ***A61K 31/453*** (2006.01)
***A61K 31/4164*** (2006.01)

(86) International application number:
**PCT/US2008/057431**

(87) International publication number:
**WO 2009/136903 (12.11.2009 Gazette 2009/46)**

# (54) STABLE OPHTHALMICAL COMPOSITION COMPRISING KETOTIFEN AND NAPHAZOLINE AND METHODS OF MAKING SAME

STABILE OPHTHALMISCHE ZUSAMMENSETZUNG MIT KETOTIFEN UND NAPHAZOLIN SOWIE HERSTELLUNGSVERFAHREN DAFÜR

COMPOSITION OPHTALMIQUE STABLE COMPRENANT DU KÉTOTIFÈNE ET DE LA NAPHAZOLINE ET SES PROCÉDÉS DE FABRICATION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.03.2007 US 689091**

(43) Date of publication of application:
**10.02.2010 Bulletin 2010/06**

(73) Proprietor: **Bausch & Lomb Incorporated**
**Rochester, NY 14604-2701 (US)**

(72) Inventors:
- **BRYANT, Roy, W.**
  **Dahlonega**
  **GA 30533 (US)**
- **PARIHAR, Ravi**
  **Atlanta**
  **GA 30307 (US)**
- **ROWE, Thomas**
  **Roswell**
  **GA 30076 (US)**
- **CABALLA, Susan**
  **Sugar Hill**
  **GA 30518 (US)**

(74) Representative: **Glas, Holger**
**Maiwald Patentanwalts GmbH**
**Elisenhof**
**Elisenstrasse 3**
**80335 München (DE)**

(56) References cited:
**WO-A-2006/047418** **WO-A-2007/025094**
**US-A1- 2002 165 254**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 150 234 B1

## Description

BACKGROUND

The present invention generally relates to stable pharmaceutical compositions and methods of making and using such compositions. In particular, the present invention relates to stable ophthalmic compositions containing antihistamines or mast cell stabilizers, and methods of making and using the same.

Ophthalmic compositions are useful for the treatment and temporary prevention of the signs and symptoms of ocular conditions, including allergic conjunctivitis, itching of the eye and redness of the eye. Methods of treating ocular conditions include administering to a human subject suffering therefrom or susceptible thereto an ophthalmic composition, for example, in the form of eye drops.

Ophthalmic compositions may also be useful for the treatment of dry eye condition, including inflammatory dry eye condition. Ophthalmic compositions may be formulated as single or multi dose units, with or without the use of a preservative, and may be manufactured by mixing various ingredients. The compositions may be packaged in single or multiple dosage forms, such as closed bottles, tubes, or other containers made from materials such as glass or plastic. In some cases, the packaging for the ophthalmic composition may be free or substantially free of antioxidant (e.g., as used in compositions described in U.S. Patent Nos. 6,455,547 and 6,576,649).

Typically, the compositions are administered as drops, with one or more drops of the composition being applied to an eye of the subject suffering from or susceptible to ocular conditions one or more times per day, although the frequency of administration of such compositions may be dependent on multiple factors, including the makeup of the particular composition and the condition for which the compositions are used.

Ophthalmic solutions may contain buffers, various surfactants, stabilizers, isotonic agents and the like which aid in making the ophthalmic compositions more comfortable to the user. Oftentimes the ophthalmic solutions contain such agents and the like to maintain a predictable level of efficacy over a predetermined or expected lifetime.

Maintenance of efficacy and stability of ophthalmic solutions may be required to meet various federal health and safety regulations, e.g., shelf life testing, sterility, etc. For example, ophthalmic solutions may be required to contain expiration dates posted on their container, which may be predicated on the stability of the active ingredients and other conditions inherent in the formulation and environmental exposures of the product. Oftentimes stabilizing agents, although effective in maintaining specific properties of the formulation, are undesirable ingredients as they may cause adverse side effects in end-users or promote the degradation of active agents in the formulation.

Of particular importance for efficacy and commercialization of ophthalmic solutions is solution stability. Solution stability may be dependent on the interactions of all compounds present in the formulation as well as temperature and pH. Ophthalmic compositions typically have a pH anywhere from 4 to 6. The pH value is generally targeted to provide a specific level or range which provides the least amount of discomfort to the end user. Conventionally, a buffer (e.g., buffers including citrates, phosphates, borates, bicarbonates, sodium salts, potassium salts, etc. or a buffer with intrinsic antimicrobial properties such as a sodium borate/boric acid buffer) is used to achieve and maintain a desired pH of the compositions, and/or an acid or base is added to adjust the pH of the compositions to the desired level. However, certain otherwise pharmaceutically effective active agents may undergo degradation when formulated in the presence of buffering agents.

WO 2006/047418 described ophthalmic compositions comprising ketotifen benzalkanium chloride and glycerol with a PH of 4.4 to 5.8. Naphazoline may be added.

It may be desirable for an ophthalmic composition to include a plurality of active agents. In such situations, it may be difficult or uneconomical to meet a particular shelf life target or federal regulatory requirements due to some instability of the combination of the active agents or other interaction, e.g., with certain buffering agents. This may be the result of some chemical reactivity or incompatibility of the compounds or salts thereof, for example, which leads to degradation of one or more of the active agents. Such degradation shortens the shelf life of the solution and may render the formulation pharmaceutically ineffective or non-compliant with federal regulatory requirements.

It is therefore desirable to formulate active agents in an ophthalmic composition wherein the efficacy of the active agents is maintained for an extended period of time. In addition, it is also desirable to provide such composition, the target specifications of the active agents of which are maintained for an extended period of time.

SUMMARY

In general, the present invention provides pharmaceutical compositions according to claim 1.

The present applicants unexpectedly have discovered that a composition of one or more active agents may be formulated at a relatively low initial pH, the active agents thereafter having exceptionally good stability in such composition.

In one aspect, the composition comprises at least an ophthalmic active agent or ingredient.

In another aspect, the composition is a topical composition.

In still another aspect, the active agent or ingredient comprises ketotifen or a salt thereof.

In yet another aspect, the composition comprises: (a) ketotifen or a salt thereof and (b) naphazoline or a salt thereof.

In a further aspect, the present invention provides a method of preparing a stabilized pharmaceutical composition, wherein the stability of at least an active ingredient of the composition is maintained for an extended period of time. The method comprises: (a) admixing a plurality of materials comprising said at least an active ingredient and a carrier to form a mixture; and (b) adjusting a pH of said mixture to less than or equal to 5 with a pH adjusting material, thereby producing the composition having said stability.

In still another aspect, the composition comprising: (a) ketotifen or a salt thereof in a concentration of from about 0.001% to about 0.2% (weight/volume or "w/v"); (b) naphazoline or a salt thereof in a concentration of from about 0.001% to about 0.2% (w/v); and (c) water.

In yet another aspect, the plurality of materials further comprises a tonicity adjusting agent.

In a further aspect, the plurality of materials further comprises a buffering agent that is capable of maintaining the pH of the composition at less than or equal to about 5.

In a still another aspect, the method comprises adjusting the pH of the composition to a value between about 4.3 and 4.8.

In a further aspect, the method produces said composition, the pH of which is maintainable between about 4.3 to about 4.8 when said composition is kept at 40 °C and 20% relative humidity ("RH") for at least 10 days.

In one embodiment, a method of preparing a stabilized ophthalmic composition is provided. The method comprises: (a) preparing a mixture comprising (1) ketotifen or a salt thereof in a concentration from about 0.001% to about 0.2% ; (2) naphazoline or a salt thereof in a concentration from about 0.001% to about 0.2%; (3) glycerol in a concentration from about 2% to 6% ; and (4) water, and (b) adjusting a pH of the ophthalmic composition to a value in a range from about 4.3 to about 4.8 to provide said stabilized ophthalmic composition, wherein said pH of the Ophthalmic composition is maintained in said range at 40 °C and 20% RH for at least 10 days.

In yet another embodiment, a method of preparing a stabilized aqueous ketotifen composition is provided. The method comprises admixing an aqueous composition comprising ketotifen or a salt thereof, with a pH adjusting agent to produce a mixture having a pH between 4.8 and 5, wherein the mixture is essentially free of buffering agents. The method further comprises allowing the pH of the mixture to adjust to between 4.3 and 4.8; thereby providing said stabilized aqueous ketotifen composition such that no more than about 10% of said ketotifen is degraded at 40 °C and 20% RH for at least 10 days.

## DETAILED DESCRIPTION

As used herein, unless otherwise specified, the concentration of a component or ingredient of a composition is represented by mass of the component or ingredient per total volume of the composition (i.e., g/mL), and is typically expressed as a percentage. For example, a concentration of 1% means 1g per 100 mL of the composition.

In general, the present invention provides pharmaceutical compositions each comprising at least an active ingredient, wherein the compositions have a low initial pH and the stability of said at least an active ingredient in the compositions is maintained for an extended period of time.

In one aspect, the composition comprises at least an ophthalmic active agent or ingredient.

In another aspect, the composition is a topical composition.

In still another aspect, the active agent or ingredient comprises ketotifen or a salt thereof.

In yet another aspect, the composition comprises: (a) ketotifen or a salt thereof; and (b) naphazoline or a salt thereof.

In a further aspect, the present invention provides a method of preparing a stabilized pharmaceutical composition, wherein the stability of at least an active ingredient of the composition is maintained for an extended period of time. The method comprises: (a) admixing a plurality of materials comprising said at least an active ingredient and a carrier to form a mixture; and (b) adjusting a pH of said mixture to less than or equal to 5 with a pH adjusting material, thereby producing the composition having said stability.

In one embodiment, the stability of at least an active ingredient of the composition is maintained for at least 10 days after the manufacture of such composition. In another embodiment, such an extended period of time is at least one month. In still another embodiment, such an extended period of time is at least two, three, four, five, six, or twelve months, or longer.

In still another embodiment, the stability of the active ingredient is maintained when less than about 20% (or alternatively, in some embodiments with other active ingredients, less than 15%, or less than 10%, or less than 5%) (by weight) of the active ingredient has degraded or changed in such period of time.

In one aspect, a composition of the present invention is an aqueous solution.

In another aspect, a composition of the present invention is an oil-in-water emulsion.

In still another aspect, a composition of the present invention is administrable to an eye as a drop and becomes more viscous after contacting an ocular environment.

In yet another aspect, a composition of the present invention is a gel.

In a further aspect, the present invention provides a method of stabilizing an ophthalmic composition. The method comprises: (a) preparing a solution of at least an ophthalmic active agent and water; and (b) adjusting a pH of the ophthalmic composition to a value of 5 or lower. In one embodiment, the method provides for solution stability. In another embodiment, the method provides an ophthalmic composition that can provide comfort to a user of the composition.

As used herein, the term "active agent" or "active ingredient" refers to a compound or composition of matter that when administered to a subject (human or animal) causes a desired pharmacologic and/or physiologic effect by local and/or systemic action. Ketotifen, a ketotifen salt, naphazoline, and a naphazoline salt are non-limiting examples of active agents that can be used to formulate ophthalmic compositions of the present invention.

As used herein, the term "break point concentration" is defined generally as the concentration of a buffering agent that is insufficient to maintain the pH of a solution comprising one or more active agents at a temperature for a given time duration. By way of example, the break point concentration of a citrate buffer for a ketotifen salt solution is the concentration of citrate that allows a decrease in the pH value of the aqueous solution when kept at 40 °C and 20% RH for at least 10 days.

As used herein, the phrase "free or substantially free of buffer agent" refers to a composition absent a buffering agent or a composition where the amount of buffering agent is less than the break point concentration of the buffer.

The pH of an aqueous ophthalmic composition comprising an active agent, alone or in combination with other ingredients may be controlled when formulated with a buffer. However, merely achieving a stable pH of an ophthalmic composition comprising an active agent, at a predetermined value, may not be sufficient to maintain the stability of the active ingredient and/or ocular comfort of the ophthalmic composition. For example, ketotifen fumarate may degrade upon storage when certain buffering agents are used. What is desirable is to provide an ophthalmic composition the pH of which does not exceed about 5 upon storage. In particular, when certain aqueous ophthalmic composition comprising ketotifen or ketotifen salts as active agent are prepared with initial pH values above 5 and stored for any appreciable amount of time, there occurs a rapid degradation of the ketotifen, a pH drift and/or increased ocular discomfort. For example, a ketotifen formulation may be manufactured at an initial pH of 5.5, with an osmolality of about 470 mOsm/kg, yet, such a formulation may substantially chemically degrade and drift to a lower pH, which may cause, among other things, unacceptable ocular irritancy in a user. Moreover, adding a buffer to the aforementioned formulation to stabilize the pH of the formulation may provide pH stability but may not provide a chemically stable solution. More likely, the presence of buffer agents may actually exacerbate the degradation and ocular discomfort of the formulation.

Buffered aqueous ophthalmic compositions comprising ketotifen or a salt thereof, initially formulated with pH values of greater than 5, maintained their starting pH values but degraded rapidly, e.g., 85% of the ketotifen in the solution initially formulated at pH 6.5 degraded after one week at 55 °C/20% RH. In contrast, the pH of unbuffered ketotifen solutions with equivalent initial pH values drifted to a lower pH value over time, in some cases, to less than 4.5, and such solutions maintained their chemical stability to a greater extent than the buffered solutions. Generally, buffered ketotifen solutions with initial pH values of 6.5, 6, 5.5, or 5 resulted in ketotifen degradation far exceeding 10 % of the initial amount present in the solution, whereas solutions with initial pH values of less than about 5 without buffer resulted in ketotifen degradation less than 10 %.

In view of the tendency of buffered solutions to promote the degradation of active agents such as ketotifen, experiments were conducted to determine whether low pH solutions of active agents could be stabilized with reduced levels of buffering agents. Experiments were conducted to determine first a break point buffer concentration for ketotifen solutions.

Thus, formulations with lower initial pH values or an amount of buffer below the break point buffer concentration were prepared and demonstrated that such formulations could provide aqueous ketotifen stability for ophthalmic compositions. On the other hand, it was recognized that lower pH values would need to be balanced with ocular comfort. Hence, formulations having an initial low pH value and/or with lower buffer concentrations were prepared and tested for drug stability, pH stability, and ocular comfort and found to satisfy both solution stability and ocular comfort. These results were generally found to be independent of additional components in the ketotifen formulation, e.g., anti-redness agents, vasoconstrictors, decongestants, viscosity-adjusting agents, tonicity-adjusting agents, and/or preservatives.

Additional experiments were conducted to determine the pH drift and ketotifen degradation as a function of initial pH, buffer agent, and concentration of buffer in ophthalmic compositions. Based on experimental data, the greatest ketotifen stability was achieved in unbuffered formulations followed by solutions having buffer concentrations below the break point concentration. From these data, it was observed that the starting point of the pH significantly affects the stability of the ketotifen in the composition.

Ketotifen or any ophthalmically acceptable ketotifen salt may be used in the method herein described, although

ketotifen fumarate is preferred. Ketotifen fumarate is represented by the following formula:

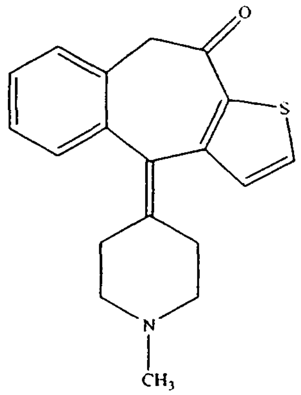

•

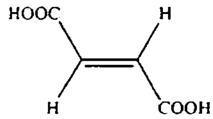

.

Ketotifen or a ketotifen salt may be present in a composition produced by a method in a concentration from about 0.001 % to about 0.2% (or alternatively, from about 0.001% to about 0.1%). In one embodiment, ketotifen or a ketotifen salt is present in a concentration from about 0.01% to about 0.05%; preferably, from about 0.01% to about 0.04%; more preferably, from about 0.02% to about 0.03%. In some embodiments, the method provides stability to compositions comprising a ketotifen or ketotifen salt in a concentration such that the concentration of ketotifen in the composition is from about 0.01% to about 0.05%; preferably, from about 0.0225% to about 0.0275%; more preferably, about 0.025%. Concentrations of ketotifen salts yielding such concentrations of ketotifen may be readily calculated; for example, using ketotifen fumarate in a concentration of about 0.0345% in the composition provides a concentration of ketotifen in the composition of 0.025%.

The Ophthalmic compositions prepared by the methods herein disclosed may include an anti-redness agent, which may relieve redness in the eye. The preferred anti-redness agent is naphazoline or an ophthalmically acceptable salt thereof such as, for example, naphazoline hydrochloride. Other anti-redness agents that may be used include, but are not limited to, tetrahydrozoline, ephedrine, phenylephrine, oxymetazoline, xylometazoline, pseudoephedrine, tramazoline, other vasoconstrictors, combinations thereof, as well as ophthalmically acceptable salts thereof (e.g., tetrahydrozoline hydrochloride).

Naphazoline hydrochloride is represented by the following formula:

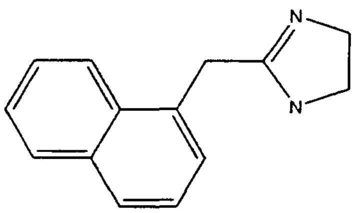

• HCl

Naphazoline or a naphazoline salt may be present in a composition produced a method of the present invention in a concentration from about 0.001 % to about 0.2% (or alternatively, from about 0.001% to about 0.1%). In one embodiment, naphazoline or a naphazoline salt is present in a composition at a concentration from about 0.01% to about 0.1%; preferably, from about 0.01% to about 0.07%; more preferably, from about 0.02% to about 0.06%. In some embodiments, the method provides stability to compositions comprising naphazoline or a naphazoline salt in a concentration such that the concentration of naphazoline in the composition is about 0.02% to about 0.05%. Concentrations of a naphazoline salt yielding such concentrations of naphazoline base may be readily calculated; for example, using naphazoline hydrochloride in a concentration of about 0.025% in the composition provides a concentration of naphazoline base in the composition of 0.021%.

In one aspect, the method herein described provides stability to pharmaceutical compositions, such as ophthalmic solutions, adjusted with tonicity agents to approximate the osmotic pressure of normal lachrymal fluids, which, as stated in U.S. Patent No. 6,274,626, is equivalent to a 2.5% solution of glycerol. Osmotic pressure, measured as osmolality, is generally about 225 to 400 mOsm/kg for conventional ophthalmic solutions.

However, in some embodiments, the pharmaceutical composition may be formulated to osmolality in the range from about 400 to about 875 mOsm/kg, for some desired purposes. In particular, such osmolality may be employed if the composition is formulated to be well tolerated by a user. For example, co-assigned U.S. Patent Application No. 2006/0148899, provides for ophthalmic solutions having osmolality from 400 to 875 mOsm/kg, which have been found

still to provide comfort to a user.

The nonionic tonicity agent is preferably glycerol, although other nonionic tonicity agents may be used such as, for example, urea, sorbitol, mannitol, propylene glycol, and dextrose. In other embodiments, glycerol is used as the nonionic tonicity agent in a concentration of from 2% to 6%, preferably from 3% to 5%, more preferably about 4 % such that the composition has an osmolality from about 200 to about 700 mOsm/kg, preferably from about 400 to about 600 mOsm/kg.

The ophthalmic compositions of the method comprising ketotifen or a ketotifen salt, an anti-redness agent, a nonionic tonicity agent, and water, may optionally include a preservative. The ophthalmic compositions may optionally include a buffer agent to maintain the pH of the composition. In a preferred embodiment, the ophthalmic composition is free or substantially free of buffer agents that would have been routinely used to achieve and/or maintain the pH of pharmaceutical compositions.

In certain embodiments, an ophthalmic composition of the present invention further comprises a carboxy-containing vinyl polymers. In one embodiment, such a polymer comprises a lightly crosslinked carboxy-containing vinyl polymer.

Crosslinked carboxy-containing polymers used in practicing this invention are, in general, well known in the art. In one embodiment, such polymers may be prepared from at least about 90% (by weight) and preferably, from about 95% to about 99.9% (by weight), based on the total weight of monomers present, of one or more carboxy-containing monoethylenically unsaturated monomers. Acrylic acid is the preferred carboxy-containing monoethylenically unsaturated monomer, but other unsaturated, polymerizable carhoxy-containing monomers, such as methacrylic acid, ethacrylic acid, β-methylacrylic acid (crotonic acid), cis-α-methylcrotonic acid (angelic acid), trans-α-methylcrotonic acid (tiglic acid), α-butylcrotonic acid, α-phenylacrylic acid, α-benzylacrylic acid, α-cyclohexylacrylic acid, β-phenylacrylic acid (cinnamic acid), coumaric acid (o-hydroxycinnamic acid), umbellic acid (p-hydroxycoumaric acid), and the like can be used in addition to or instead of acrylic acid.

Such polymers may be crosslinked by a polyfunctional crosslinking agent, preferably a difunctional crosslinking agent. The amount of crosslinking should be sufficient to form insoluble polymer particles, but not so great as to unduly interfere with sustained release of the medicament. Typically, the polymers are only lightly crosslinked. Preferably, the crosslinking agent is contained in an amount of from about 0.01% to about 5% (by weight); more preferably, from about 0.1% to about 5% (by weight), and more preferably from about 0.2% to about 1% (by weight), based on the total weight of monomers present. Included among such crosslinking agents are non-polyalkenyl polyether difunctional crosslinking monomers such as divinyl glycol; 2,3-dihydroxyhexa-1,5-diene; 2,5-dimethyl-1,5-hexadiene; divinylbenzene; N,N-diallylacrylamide; N,N-diallymethacrylamide and the like. Also included are polyalkenyl polyether crosslinking agents containing two or more alkenyl ether groupings per molecule, preferably alkenyl ether groupings containing terminal $CH_2=C<$ groups, prepared by etherifying a polyhydric alcohol containing at least four carbon atoms and at least three hydroxyl groups with an alkenyl halide such as allyl bromide or the like, e.g., polyallyl sucrose, polyallyl pentaerythritol, or the like; see, e.g., U.S. Patent 2,798,053. Diolefinic non-hydrophilic macromeric crosslinking agents having molecular weights of from about 400 to about 8,000, such as insoluble di- and polyacrylates and methacrylates of diols and polyols, diisocyanate-hydroxyalkyl acrylate or methacrylate reaction products of isocyanate terminated prepolymers derived from polyester diols, polyether diols or polysiloxane diols with hydroxyalkylmethacrylates, and the like, can also be used as the crosslinking agents; see, e.g., U.S. Patents 4,192,827 and 4,136,250.

The crosslinked polymers may be made from a carboxy-containing monomer or monomers as the sole monoethylenically unsaturated monomer present, together with a crosslinking agent or agents. Preferably, the polymers are ones in which up to about 40%; and more preferably, from about 0.0001% to about 20% by weight, of the carboxy-containing monoethylenically unsaturated monomer or monomers has been replaced by one or more non-carboxyl-containing monoethylenically unsaturated monomer or monomers containing only physiologically and ophthalmologically innocuous substituents, including acrylic and methacrylic acid esters such as methyl methacrylate, ethyl acrylate, butyl acrylate, 2-ethylhexylacrylate, octyl methacrylate, 2-hydroxyethylmethacrylate, 3-hydroxypropylacrylate, and the like, vinyl acetate, N-vinylpyrrolidone, and the like; see U.S. Patent 4,548,990 for a more extensive listing of such additional monoethylenically unsaturated monomers. Particularly preferred polymers are lightly crosslinked acrylic acid polymers wherein the crosslinking monomer is 2,3-dihydroxyhexa-1,5-diene or 2,3-dimethylhexa-1,5-diene. Preferred commercially available polymers include polycarhophil (Noveon AA-1) and Carbopol®.

The crosslinked polymers used in practicing this invention are preferably prepared by suspension or emulsion polymerizing the monomers, using conventional free radical polymerization catalysts, to a dry particle size of not more than about 50 μm in equivalent spherical diameter; e.g., to provide dry polymer particles ranging in size from about 1 to about 30 μm, and preferably, from about 3 to about 20 μm, in equivalent spherical diameter. Using polymer particles that were obtained by mechanically milling larger polymer particles to this size is preferably avoided. In general, such polymers will have a molecular weight which has been variously reported as being from about 250,000 to about 4,000,000, and from 3,000,000,000 to 4,000,000,000.

In a preferred embodiment of the invention, the particles of crosslinked polymer are monodisperse, meaning

that they have a particle size distribution such that at least 80% of the particles fall within a 10 μm hand of major particle size distribution. More preferably, at least 90% and most preferably at least 95%, of the particles fall within a 10 μm band of major particle size distribution. Also, a monodisperse particle size means that there is no more than 20%, preferably no more than 10%, and most preferably no more than 5% particles of a size below 1 μm. The use of a monodispersion of particles will give maximum viscosity and an increased eye residence time of the ophthalmic medicament delivery system for a given particle size. Monodisperse particles having a particle size of 30 μm and below arc most preferred. Good particle packing is aided by a narrow particle size distribution.

In one embodiment, the ophthalmic composition comprises a polymer component that consists essentially of one or more of the above-described crosslinked carboxy-containing polymers. This means that no additional polymers are present in the composition that would significantly affect the medicament release profile. Polymers and oligomers used as excipients, carriers, demulcents, or other non-medicament-interactive functions are still included within the composition so long as the medicament release profile is not significantly altered. However, in this embodiment no polymer particles (water insoluble polymers) which materially affect release e.g., a cationic exchange resin) are present in addition to the crosslinked carboxy-containing polymers, and typically no other polymers (soluble or insoluble) of any kind are present in the composition.

When such crosslinked carboxy-containing polymer is present in an ophthalmic composition of the present invention, it is generally present in an amount ranging from 0.5 to 2%; preferably, from about 0.5% to about 1.2% (w/v); and more preferably, from about 0.6 to about 0.9% (w/v).

The ophthalmic compositions may include an acid or base to adjust the pH of the composition. The method is useful for the stabilization of ophthalmic solutions that have a pH value initially adjusted such that the pH value of the ophthalmic composition thereafter is maintainable between about 4.3 and about 4.8 at least for 10 days at 40 °C and 20% relative humidity. The solutions may be adjusted to any pH value such that the pH value of the ophthalmic composition thereafter is between about 4.3 and about 4.8. The solutions preferably may be initially adjusted to have a pH value above 4.5 or below 5.0. Preferably, the adjusted pH value is higher than the thereafter pH value of the composition. Most preferred is an initially adjusted solution having a pH value of about 4.8.

Typically, only small amounts of an acid or base will be needed to adjust the initial pH of the solution. By way of example, an acid and base suitable for adjusting the pH are hydrochloric acid and sodium hydroxide. Fumaric acid or fumaric acid/sodium fumarate may also be suitable to adjust the pH of the solution. A buffering agent (e.g., buffers including citrates, phosphates, borates, bicarbonates, sodium salts, potassium salts, etc.; or a buffer with intrinsic antimicrobial properties such as a sodium borate/boric acid buffer) may be used provided that the breakpoint buffer concentration is not exceeded. If a buffering agent is used, it is further preferred that no more than 10% of the concentration of any active agents in the composition is degraded, for example, at 40 °C and 20% RH for at least 10 days. Preferably, the ophthalmic solution is free or substantially free of buffering agent.

In one embodiment, the method is useful for the stabilization of compositions that include a preservative. In another embodiment, the method is useful for the stabilization of compositions that does not include a preservative. A preservative is preferred when the composition is packaged for multidose units, but may be absent from the composition if desired (e.g., in single dose units of the composition). Any preservative may be used with the compositions. Preservatives that may be used include Polyquad preservative (Alcon); perborate (e.g., sodium perborate from Ciba); Purite preservative (stabilized chlorine dioxide) (Allergan); other quaternary ammonium compounds such as benzalkonium chloride; alkyl-mercury salts of thiosalicylic acid such as, for example, thiomersal, phenylmercuric nitrate, phenylmercuric acetate, and phenylmercuric borate; parabens such as, for example, methylparaben or propylparaben; alcohols such as, for example, chlorobutanol, benzyl alcohol, and phenyl ethanol; guanidine derivatives such as, for example, chlorhexidine or polyhexamethylene biguanide; and the like. When a preservative is used in the composition, the preservative is typically provided in a concentration of about 0.005% to 0.02%, preferably 0.01%, although other concentrations may be used.

In one embodiment, the method herein described provides for preparing a stabilized ophthalmic composition. The method comprises the steps of preparing an aqueous solution consisting essentially of ketotifen or a ketotifen salt in a concentration of from about 0.01% to about 0.05%; naphazoline or a naphazoline salt in a concentration of from about 0.01% to about 0.1 %; glycerol; benzalkonium chloride; and water. In one embodiment, the aqueous solution aqueous solution consists essentially of ketotifen or a ketotifen salt in a concentration of from about 0.01% to about 0.05%; naphazoline or a naphazolinc salt in a concentration of from about 0.01% to about 0.1%; glycerol; benzalkonium chloride; water; and a buffering agent. The method provides for adjusting the pH value of the aqueous solution to less than or equal to about 5 by adding a pH adjusting agent, and providing a stabilized ophthalmic composition where the pH value of the ophthalmic composition is maintainable between about 4.3 to about 4.8 when kept at 40 °C and 20% RH for at least 10 days.

As used herein, "maintainable" and grammatical equivalents thereof refers generally to a value of a property of a composition that is capable of being determined, that stays within a defined range or meets a specified target value during an interval of time associated with the storage of the composition. By way of example, an ophthalmic composition

stored at 40 °C and 20% RH for at least 10 days that is determined to have a value corresponding to a defined range or specified target value, that value would be "maintainable." An example of values that may be maintainable in accordance with the method herein described includes, without limitation, pH, ocular comfort and concentrations of the portion of an active agent that has not degraded or changed.

Concentrations of one or more active agents may change during storage. As used herein, "during storage" refers to any interval of time associated with the preparation, handling, sterilizing, transporting and distributing or marketing of the composition. The composition may be in whatever container or form as may be desirable. During storage also includes accelerated aging testing, or other testing as may be required by state and federal regulation, e.g., Food and Drug Administration (FDA) rules, regulations and protocols. By way of example, during storage includes 40 °C and 20% relative humidity for at least 10 days.

If the amount of active agent falls below a predetermined level the composition may not provide the desired pharmaceutical effect that was intended. Furthermore, a shelf life of an ophthalmic composition may be correlated to or predicted by the amount of initial concentration of active agent(s) remaining at any given interval of time after formulating, packaging, sterilizing, etc. In one embodiment of a method of stabilizing an ophthalmic composition, a ratio of a determined concentration of an active agent in the composition after an interval of time from when the composition is formulated to an initial concentration of the active agent in the composition is provided. Generally the ratio may be expressed in percentage that has degraded. For example, an active agent with an initial concentration of 10 μg/L that degrades, for example, during storage to 8 μg/L of active agent would have 20% of the active agent degraded. Concentration of an active agent in a composition may be determined by a HPLC method. The initial concentration may correspond to a stated concentration of active agent on a label affixed to the container, box or insert provided with the ophthalmic composition, e.g, "label claim," or to a pharmaceutically effective concentration of active agent.

Degradation of active agent refers generally to an active agent that has changed chemically such that a pharmaceutical property of the active agent is reduced or eliminated. Methods of determining the amount of degradation of active agents and concentrations of initial active agent remaining after an interval of time has elapsed are generally known. For example, an active agent that is detectable by a detection method generally used to determine a concentration of the active agent may be used to determine whether the concentration of the active agent has decreased relative to its initial formulated concentration. The detection method may only measure the concentration of active ingredient.

By adjusting the pH value of an ophthalmic solution such that the pH value of the ophthalmic composition is maintainable between about 4.3 and about 4.8 for example, at 40 °C and 20% RH for at least 10 days, it may be possible to substantially eliminate the need for a buffer agent, or it may provide for the use of very low concentrations of buffering agents. Providing ophthalmic compositions free or essentially free of buffer improves ocular comfort of the composition for the user. The method herein described may be useful for providing acceptable ocular comfort ophthalmic compositions comprising ketotifen as well as compositions comprising ketotifen in combination with anti-redness agents, for example, naphazoline or naphazoline salts.

As used herein, ocular comfort refers to an effect of an ophthalmic composition on a user upon contact of the composition with an ocular space of the user. Ocular comfort is determined by a user responding to the introduction of drops of a composition into the eye of the user. By way of example, the response may be graded on a numerical scale, from 1 to 10, 1 representing mostly discomfort, and 10 representing mostly comfort or the response may be an indication that the ocular comfort is acceptable or unacceptable. In certain embodiments, the methods herein disclosed can be useful for the stabilization of compositions that are also free or substantially free of stabilizers such as ethylene diamine tetraacetic acid (EDTA) and salts thereof. Dequest, and Desferal (e.g., as used in compositions described in U.S. Patents 6,776,982 and 6,468,548); polymers comprising chitosan (e.g., as used in compositions described in U.S. Patent Application No. 2003/0031718); linear polysaccharide compounds such as hyaluronic acid compounds (e.g., as used in compositions described in International Publication No. WO 02/100437); biocompatible polymers/thickeners such as polyoxyethylene-polyoxypropylene copolymers; antioxidants; and/or active agents other than ketotifen or naphazoline. For example, the methods herein disclosed may be useful for the stabilization of compositions consisting essentially of ketotifen or a ketotifen salt and anti-redness agent, a nonionic tonicity agent, and water, free or substantially free of these aforementioned components.

Alternatively, the methods herein disclosed are useful for the stabilization of an ophthalmic composition comprising ketotifen or a ketotifen salt, naphazoline or naphazoline salt, a nonionic tonicity agent, and water, and a preservative, and optionally an acid, base or buffer agent to adjust the pH of the composition.

The methods herein disclosed may be useful for the stabilization of an ophthalmic composition consisting of ketotifen or a ketotifen salt, naphazoline or naphazoline salt, a nonionic tonicity agent, and water free or substantially free of buffer agents. The nonionic tonicity agent may be present in a concentration such that the composition has an osmolality from 200 to 700 mOsm/kg, preferably from 400 to 600 mOsm/kg. The nonionic tonicity agent may be glycerol. The concentration of glycerol may be about 2% to about 6%, The concentration of glycerol preferably may be about 3% to about 5%, and most preferably, 4%.

The methods herein disclosed may be useful for the stabilization of an ophthalmic composition of ketotifen or

a ketotifen salt such that no more than about 10% of the ketotifen or the ketotifen salt is degraded at 40 °C and 20% RH for at least 10 days. The methods herein disclosed also may be useful for the stabilization of an ophthalmic composition of naphazoline or naphazoline salt such that no more than about 5% of the 5% of the naphazoline or the naphazoline salt is degraded at 40 °C and 20% RH for at least 10 days. Further, the methods herein disclosed may be useful for the stabilization of an ophthalmic composition of ketotifen and naphazoline (or their salts) such that no more than about 10% of the ketotifen or the ketotifen salt and no more than about 5% of the naphazoline or the naphazoline salt when combined together are degraded at 40 °C and 20% relative humidity for at least 10 days.

EXAMPLES

The following examples are illustrative of the embodiments of the present invention and are not to be interpreted as limiting or restrictive. Notwithstanding that the numerical ranges and parameters setting forth the broad scope of the invention are approximations, the numerical values set forth in the specific examples are reported as precisely as possible. Any numerical value, however, inherently contains certain uncertainties, as expressed by the standard deviation found in its respective measurements (e.g., pH), where such standard deviation can be determined or estimated. By way of example, a pH value is to be regarded as to be within a range of ± 0.2.

In one example, a method of stabilizing an ophthalmic composition is provided as follows. The method comprises preparing a solution comprising ketotifen fumarate in a concentration of from about 0.01% to about 0.05%, naphazoline hydrochloride in a concentration of from about 0.01 % to about 0.1 %, a glycerol concentration such that the solution has an osmolality of from 200 to 700 mOsm/kg (milliosmole/kg) and water. A preservative may be added. The concentration of preservative may be about 0.01 %, however, lower or higher concentrations may be used, in appropriate cases. The preservative can be benzalkonium chloride. The solution is prepared by contacting the salts with the water.

In another example, a method of preparing a stabilized ophthalmic composition is provided as follows. The method comprises preparing a composition consisting essentially of ketotifen fumarate in a concentration of from about 0.01 % to about 0.05%, naphazoline hydrochloride in a concentration of from about 0.0 1 % to about 0.1 %, a glycerol concentration such that the composition has an osmolality of from 200 to 700 mOsm/kg, benzalkonium chloride in a concentration of about 0.01%, a buffering agent; and water. The method further comprises adjusting a pH value of the composition to less than about 5. By adjusting the pH of the ophthalmic composition the pH value is maintainable between about 4.3 and about 4.8 at 40 °C and 20% RH for at least 10 days.

In still another example, a method of stabilizing an ophthalmic composition is provided. The method comprises preparing a composition comprising a ketotifen base in a concentration of about 0.025%, a naphazoline base in a concentration of about 0.02% to about 0.05%, glycerol in a concentration of about 2% to 6%, and water. The pH value of the composition is adjusted, where the pH value is maintainable between about 4.3 and about 4.8 at 40 °C and 20% RH for at least 10 days. The osmolality of the composition is from about 400 to about 600 mOsm/kg. The composition may further comprise a citrate buffer in a concentration of about 0.002M or less. The composition may further comprise benzalkonium chloride in a concentration of about 0.01%.

In yet another example, a method of stabilizing an ophthalmic composition is provided. The method comprises adjusting a pH value of the ophthalmic composition to less than about 5, the composition comprising a ketotifen or a ketotifen salt, an anti-redness agent, and water, where the ketotifen or the Ketotifen salt is degraded less than 10% at 40 °C and 20% RH for at least 10 days. The ketotifen salt may be ketotifen fumarate. The ketotifen fumarate may be present in a concentration of from about 0.01% to about 0.05%. The anti-redness agent may be a naphazoline salt, preferably naphazoline hydrochloride. The naphazoline hydrochloride may be present in a concentration of from about 0.01% to about 0.1%. In the above example, glycerol may be present, preferably in a concentration of about 2% to about 6%. The composition may further comprise a citrate buffer in a concentration of about 0.002M or less provided that no more than about 10% of the ketotifen degrades after at least 10 days at 40 °C and 20% RH. The composition may further comprise benzalkonium chloride in a concentration of about 0.005% to about 0.02%.

Formulations comprising ketotifen and naphazoline free or substantially free of buffering agents were prepared with adjusted initial pH values. Controls comprising ketotifen and naphazoline with various buffering agents were also prepared with adjusted initial pH ranges. The formulations and the control samples were tested for their stability at various temperatures and RHs. The pH of the formulations and the control samples were tested using a Fisher Acumet pH meter. Degradation analysis of the active ingredients in the formulations was performed using HPLC using control samples for ketotifen and naphazoline. The HPLC procedure utilized a Xterra 3.5 um C 18, 150 x 2.1 mm ID, column (Waters, part # OOF-4114-DO) or equivalent. The gradient conditions consisted of mobile phase A comprising a 60:40 v/v mixture of a solution of 2 mL triethylamine in 2000 mL water and a solution of 2 mL triethylamine in methanol, respectively, and a mobile phase B comprising 2 mL triethylamine in methanol. Mobile phase gradient conditions were as summarized in Table I.

Table I

| Mobile Phase Gradient Conditions for Ketotifen HPLC Assay | | |
|---|---|---|
| Time (min) | Mobile Phase A (%v/v) | Mobile Phase B (%v/v) |
| 0 | 100 | 0 |
| 10 | 100 | 0 |
| 20 | 50 | 50 |
| 35 | 50 | 50 |
| 36 | 100 | 0 |
| 45 | 0 | 0 |

Detection of the active ingredients was achieved with a variable wavelength ultraviolet detector. Degraded sample controls were prepared by pipetting 10 mL of the above solutions and combining with 0.1 mL sodium hydroxide solution (20 w/v%) followed by heating for 30 minutes at 70 °C. After cooling, the pH was adjusted to 4.8 ± 0.5 with dilute HCl solution.

Ketotifen fumarate concentration was calculated using equation (I):

$$\text{mg/mL ketotifen fumarate} = \frac{A_{Ketspl}}{Aavg_{Ketstd}} \times \text{Std Diln} \times P_{std} \times M_{st} \quad \text{(I)}$$

where $A_{Ketspl}$ = area of ketotifen in sample chromatogram; Aavg $_{Kelstd}$ = average area of ketotifen from bracketed standards; Std Diln = standard dilutions; $P_{std}$ = purity of standard expressed as a decimal; and $M_{st}$ = Moisute factor (e.g., (100-limit of detection/100).

Naphazoline hydrochloride concentration was calculated using equation (II):

$$\text{mg/mL naphazoline hydrochloride} = \frac{A_{Naphspl}}{Aavg_{Naphstd}} \times \text{Std Diln} \times P_{std} \quad \text{(II)}$$

where $A_{Naphspl}$ = area of naphazoline in sample chromatogram; Aavg $_{Naphstd}$ = average area of naphazoline from bracketed standards; Std Diln = standard dilutions; and $P_{std}$ = purity of standard expressed as a decimal.

The data is summarized in Tables II-V. Table II depicts experimental results of storage at 55 °C/20% RH for four weeks of buffered control sample solutions A-F and unbuffered formulation G. The samples at pH 6.5 and pH 6 (A, B, D and E) were dropped from the stability testing after 1 week, since assay results indicated that ketotifen concentration had dropped to less than 15% of the initial ketotifen concentration. These buffered samples (A, B, D and E), however, maintained their initial pH values for the 1 week.

TABLE II

| Sample | Composition | Buffer | Conc. of Buffering Agent M | Initial pH | Final pH | %Ketotifen detected | %Naphazoline detected |
|---|---|---|---|---|---|---|---|
| | | | | | | | |
| A | Ketofen/Naphazoline | citrate | 0.02 | 6.5 | ND | <15 | ND |
| B | Ketotifen/Naphazoline | citrate | 0.02 | 6.0 | ND | <15 | ND |
| C | Ketotifen/Naphazoline | citrate | 0.02 | 5.5 | 5.5 | 60.0 | ND |
| | | | | | | | |
| D | Ketotifen/Naphazoline | phosphate | 0.02 | 6.5 | ND | <15 | ND |
| E | Ketotifen/Naphazoline | phosphate | 0.02 | 6.0 | ND | <15 | ND |

(continued)

| Sample | Composition | Buffer | Conc. of Buffering Agent M | Initial pH | Final pH | %Ketotifen detected | %Naphazoline detected |
|---|---|---|---|---|---|---|---|
| F | Ketotifen/Naphazoline | phosphate | 0.02 | 5.5 | 5.5 | 60.0 | ND |
| | | | | | | | |
| G | Ketotifen/Naphazoline | None | 0 | 5.5 | 4.5 | 85.0 | 86.0 |
| | | | | | | | |
| Stability Study Conditions: 55 °C/20% RH, 4 weeks<br>ND = not determined | | | | | | | |

Table II indicates that the initial pH value of the buffered solutions C and F remained at their initial pH values; however, ketotifen assay results indicated that the ketotifen concentration had dropped to 60% of the initial ketotifen concentration. In contrast, for the unbuffered formulation sample G, which decreased in initial pH value after about 1 week, ketotifen assay results indicated that the ketotifen concentration had dropped to only 85% of the initial ketotifen concentration. Naphazoline assay results for the unbuffered formulation sample G indicated that the naphazoline concentration had dropped to 86% of the initial naphazoline concentration. Thus, the unbuffered formulation sample G maintained a higher ketotifen and naphazoline concentration with a decrease in initial pH of 5.5 to a pH value of 4.5 compared to the buffered controls. The data of Table II indicates that there is little distinguishable difference between the type of buffer used (citrate or phosphate) and the extent of degradation of active agent observed.

TABLE III

| Sample | Composition | Buffer | Conc. of Buffering Agent (M) | Initial pH | Final pH | % Ketotifen detected | % Naphazoline detected |
|---|---|---|---|---|---|---|---|
| H | Ketotifen/Naphazoline | citrate | 0.005 | 5.5 | 5.5 | 66.0 | 79.0 |
| I | Ketotifen/Naphazoline | citrate | 0.005 | 5.0 | 5.0 | 70.0 | 83.0 |
| | | | | | | | |
| J | Ketotifen/Naphazoline | phosphate | 0.005 | 5.5 | 5.5 | 68.0 | 78.0 |
| K | Ketotifen/Naphazoline | phosphate | 0.005 | 5.0 | 5.0 | 73.0 | 81.0 |
| | | | | | | | |
| L | Ketotifen/Naphazoline | None | 0 | 5.5 | 4.5 | 79.0 | ND |
| | | | | | | | |
| Stability Study Conditions: 55 °C/20% RH 4 weeks<br>ND = not determined | | | | | | | |

Table III depicts experimental data directed to stabilizing ketotifen/naphazoline solutions formulated without buffer versus controls having reduced concentration of buffer. Thus, four buffered control samples (H-K) and an unbuffered formulation (L) were stability tested at 55 °C/20% RH. The controls contained reduced levels of buffer (25% less than the previous controls) and initial pH values of 5.5 and 5. The unbuffered formulation L was adjusted to an initial pH value of 5.5.

The data of Table III indicates that all of the buffered controls maintained their respective pH values throughout the stability study. Unbuffered formulation L decreased in pH value from an initial pH value of 5.5 to a value of 4.5. Assay results of % initial ketotifen and naphazoline concentrations of the post-stability tested compositions showed that the greatest amount of degradation occurred in the buffered samples. No significant difference in degradation of active ingredients was observed between citrate and phosphate buffer at the pH values tested. Decreasing either the citrate or phosphate buffer concentration reduced the total degradation of ketotifen.

Naphazoline assay recoveries for the lower concentration phosphate and citrate buffered controls were marginally improved over control samples with higher concentration of buffer when of the initial pH value was adjusted lower.

TABLE IV

| Sample | Composition | Bluffer | Conc. of Buffering Agent (M) | Initial pH | Final pH | %Ketotifen detected | %Naphazoline detected |
|---|---|---|---|---|---|---|---|
| M | Ketotifen/Naphazoline | citrate | 0.002 | 4.8 | 4.7 | 72.0 | 99.0 |
| N | Ketotifen/Naphazoline | citrate | 0.001 | 4.8 | 4.5 | 77.0 | 96.0 |
| | | | | | | | |
| O | Ketotifen/Naphazoline | phosphate | 0.004 | 4.8 | 4.7 | 71.0 | 99.0 |
| P | Ketotifen/Naphazoline | phosphate | 0.002 | 4.8 | 4.5 | 65.0 | 98.0 |
| | | | | | | | |
| Q | Ketotifen/Naphazoline + 2.2% Glycerol | None | 0 | 4.8 | 4.5 | 92.0 | 99.7 |
| R | Ketotifen/Naphazoline + 4% Glycerol | None | 0 | 4.8 | 4.4 | 92.0 | 99.7 |
| S | etotifen + 2.2% Glycerol | None | 0 | 4.8 | 4.4 | ND | ND |
| T | Ketotifen/Naphazoline | None | 0 | 4.5 | 4.0 | 97.4 | 102.7 |
| U | Ketotifen/Naphazoline | None | 0 | 4.0 | 3.7 | 98.0 | 99.9 |
| | | | | | | | |
| Stability Study Conditions: 55 °C/20% RH, 4 weeks<br>ND = not determined | | | | | | | |

Table IV depicts additional buffered controls M-P with lower concentrations of buffer and unbuffered formulations Q-U. The buffer concentrations were chosen so as to find a break point concentration where the concentration of buffer would not be able to maintain the initial pH value over time. The buffered controls M-P and unbuffered formulations Q-U were adjusted to have initial pH values of 4.8.

As indicated in Table IV, at 55 °C, the pH values for the buffered controls M-P decreased from their initial value over the four weeks. The data indicate that the greatest decrease of initial pH value for the buffered control samples was observed for control sample O (0.001M citrate; final pH value 4.5) and control sample P (0.002M phosphate; final pH value 4.5), which indicated that these concentrations represented the break point concentration for the respective buffering agents for the ketotifen/naphazoline formulation. At 0.002M citrate and 0.004M phosphate, concentrations exceeding the break point concentration, the data indicated that these formulations experienced levels of ketotifen degradation of greater than 25% or more. Other buffer systems besides citrate buffer and phosphate buffer are envisaged as providing pH stability and solution stability to active agent ophthalmic formulations such as ketotifen and ketotifen/naphazoline formulations provided that the break point of the particular buffer solution is determined and not exceeded.

Table IV also contains stability data obtained from unbuffered ketotifen and unbuffered ketotifen-naphazoline solutions comprising nonionic tonicity agents. Solutions of unbuffered ketotifen-naphazoline with 2.2 % glycerin (sample Q), unbuffered ketotifen-naphazoline 4% glycerin (sample R) and unbuffered ketotifen with 2.2% glycerin (sample S) were prepared with adjusted low pH values and tested as described above. These formulations exhibited decreased pH over time as previously observed and maintained greater than 90% initial ketotifen concentration.

Two additional unbuffered formulations were prepared and tested as described above. Thus, samples T and U were formulated with adjusted initial pH values of 4.5 and 4.0, respectively, and their stability tested at 55 °C for 4 weeks.

TABLE V

| Sample | Composition | Buffer | Conc. of Buffering Agent (M) | Initial pH | Final pH | %Ketotifen detected | %Naphazoline detected |
|---|---|---|---|---|---|---|---|
| V | Ketotifen/Naphazoline | citrate | 0.002 | 4.8 | 4.8 | 93.1* | 100* |

(continued)

| Sample | Composition | Buffer | Conc. of Buffering Agent (M) | Initial pH | Final pH | %Ketotifen detected | %Naphazoline detected |
|---|---|---|---|---|---|---|---|
| W | Ketotifen/Naphazoline | citrate | 0.001 | 4.8 | 4.6 | 95.1* | 100* |
| | | | | | | | |
| X | Ketotiten/Naphazoline | phosphate | 0.004 | 4.8 | 4.7 | 87* | 99.6* |
| Y | Ketotifen/Naphazoline | phosphate | 0.002 | 4.8 | 4.7 | 87* | 99.2* |
| | | | | | | | |
| AA | Ketotifen/Naphazoline + 2.2% Glycerol | None | 0 | 4.8 | 4.3 | 96.5 | 101.1 |
| BB | Ketotifen/Naphazoline + 4% Glycerol | None | 0 | 4.8 | 4.4 | 97.0 | 101.5 |
| | | | | | | | |
| Stability Study Conditions: 40 °C/ 20% RH for 1 week<br>* Determined after one month | | | | | | | |

As shown in Table V, the initial pH values of the unbuffered formulations AA and BB decreased while the initial concentrations of active agents did not decrease significantly. In contrast, the buffered controls maintained their pH values, but the initial concentrations of active agents decreased significantly.

For the unbuffered nonionic tonicity added formulations Q, R, AA and BB the pH values decreased after stability testing from their initially adjusted pH values. The results found in Tables IV and V indicate that unbuffered solutions containing nonionic tonicity agents, such as glycerol, as in samples Q, R, AA and BB, will also maintain ketotifen and/or naphazoline stability during testing or storage as compared to the buffered controls.

Although, the degradation of ketotifen and naphazoline were reduced or eliminated, the decrease in initial pH values for samples T and U to values of 4.0 and 3.7, respectively, were observed. At these pH values are not be recommended, as the comfort of more sensitive users may be impaired.

As shown in Tables II-V, the results of stability testing of the unbuffered formulations, with or without nonionic tonicity agent indicate lower initial pH values of solutions comprising active agents, as in the method herein described, provide a better stability profile for ophthalmic solutions. This applies to ophthalmic solutions comprising a plurality of active agents, e.g., Ketotifen/Naphazoline. Therefore, the methods herein described provide for greater efficacy and longer shelf life of ocular drug products.

While the buffered formulations tested in the foregoing experiments provided the formulations with a stable pH, they yielded faster degradation of the ketotifen and/or naphazoline. However, the degradation of ketotifen and/or naphazoline could be reduced to about less than ten percent after one month at 40 °C and 50 °C, even in the presence of 0.002 M or less citrate buffer or 0.004 M or less phosphate buffer with the present invention. The data presented herein demonstrates that by adjusting the pH value of a ketotifen fumarate/naphazoline hydrochloride solution longer shelf life and a significantly reduced degradation of ketotifen and/or naphazoline actives are provided. Based on the data described above, it is envisaged that the improvement in stability for the ketotifen/naphazoline solution can be extrapolated to methods where an initial pH value is adjusted to a range of about 4.5 to about 5.

Representative samples of the various formulations described above were tested for ocular comfort. Typically, the testing studies involve placing a drop of one formulation in one eye and a drop of a second formulation in the other eye of the subject. Subjects evaluated ocular comfort immediately after each drop and two minutes later. The subjects indicated ocular comfort as acceptable or not acceptable. The subjects were unaware as to the identity of the formulations.

Buffered and unbuffered formulations as described above were tested for ocular comfort. Unbuffered formulations were preferred over buffered formulations with regard to ocular comfort as determined by test subjects. Phosphate buffered formulations were preferred over citrate buffered formulations in the pH range of 5.6 to 5.8 with regard to ocular comfort as determined by test subjects.

In subsequent studies of ocular comfort with solutions having pH values of 4 to 4.8, it was observed that unbuffered solutions with these pH values were also acceptable with regard to ocular comfort as determined by test subjects. Extrapolation of this trend in ocular comfort and the nature of the buffer system to pH values of 4.8 to 5 are therefore envisaged, e.g., unbuffered > phosphate > citrate buffer below the break point concentration. Thus, within a range of initial pH values of 4 to 5, both the stability of the actives and the ocular comfort of ophthalmic solutions may

be maximized using the methods herein described. The pH value of the ophthalmic composition may be further adjusted as needed or desired after storage, sterilization, etc., or prior to use.

In one aspect, the present invention provides a pharmaceutical composition that comprises ketotifen or a ketotifen salt, which is present at a concentration from about 0.001% to about 0.2% (w/v) (or alternatively, from about 0.001 to about 0.1 % (w/v), or from about 0.005 to about 0.1% (w/v), or from about 0.05 to about 0.05% (w/v)), wherein a pH of the composition remains at less than 5 at 40 °C and 20% RH for at least 10 days. In certain other embodiment, the pH of the composition remains in a range from about 4.3 to about 5 (or alternatively, from about 4.3 to about 4.8) at 40 °C and 20% RH for at least 10 days.

In certain other embodiments, a pharmaceutical composition consists essentially of: (a) ketotifen or a ketotifen salt, which is present at a concentration from about 0.001% to about 0.2% (w/v) (or alternatively, from about 0.001 to about 0.1% (w/v), or from about 0.005 to about 0.1% (w/v), or from about 0.05 to about 0.05% (w/v)); (b) naphazoline or a naphazoline, which is present at a concentration from about 0.001% to about 0.2% (w/v) (or alternatively, from about 0.001 to about 0.1% (w/v), or from about 0.005 to about 0.1% (w/v), or from about 0.05 to about 0.05% (w/v)); and (c) a pharmaceutically acceptable carrier; wherein a pH of the composition remains at less than 5 at 40 °C and 20% RH for at least 10 days.

In still certain other embodiment, a pharmaceutical composition consists essentially of: (a) ketotifen or a ketotifen salt, which is present at a concentration from about 0.001 % to about 0.2% (w/v) (or alternatively, from about 0.001 to about 0.1% (w/v), or from about 0.005 to about 0.1% (w/v), or from about 0.05 to about 0.05% (w/v)); (b) naphazoline or a naphazoline, which is present at a concentration from about 0.001% to about 0.2% (w/v) (or alternatively, from about 0.001 to about 0.1% (w/v), or from about 0.005 to about 0.1% (w/v), or from about 0.05 to about 0.05% (w/v)); (c) a tonicity-adjusting agent; and (d) a pharmaceutically acceptable carrier; wherein a pH of the composition remains at less than 5 at 40 °C and 20% RH for at least 10 days. In certain aspects, the tonicity-adjusting agent is present at a concentration such that the osmolality of the composition is in the range from about 200 to about 700 mOsm/kg (or alternatively, from about 220 to about 600 mOsm/kg, or from about 250 to about 400 mOsm/kg).

In still certain other embodiments, at least 90% of each of said ketotifen, ketotifen salt, naphazoline, and naphazoline salt, when present, remains in the composition after storage at 40 °C and 20% RH for at least 10 days.

In further embodiments, the composition is an aqueous solution, an oil-in-water emulsion, a dispersion, a gel, or a gelable formulation.

In still further embodiments, the composition has a viscosity in a range from about 5 to about 10,000 mPa.s (or centipoises). Alternatively, the viscosity is in a range from about 5 to about 1,000 mPa.s.

A composition of the present invention can be used to treat, ameliorate, or reduce a condition resulting from allergy. For example, a composition of the present invention can be applied topically to treat, ameliorate, or reduce the severity of, allergic conjunctivitis or symptoms thereof, such as pink eye, itchy eye, or combinations thereof. A composition of the present invention may be applied to the ocular surface in the form of eye drops, in one or more drops once per day, twice per day, or three times or more per day.

In another embodiment, a composition of the present invention can be formulated to be used topically for dermatological applications to treat, ameliorate, or reduce allergic symptoms.

As used herein, "comprising," "including," "containing," "characterized by," and grammatical equivalents thereof are inclusive or open-ended terms that do not exclude additional, unrecited elements or method steps. "Comprising" is to be interpreted as including the more restrictive terms "consisting of and "consisting essentially of." As used herein, "consisting of"' and grammatical equivalents thereof exclude any element, step, or ingredient not specified in the claim.

As used herein, "consisting essentially of" and grammatical equivalents thereof limit the scope of a claim to the specified materials or steps and those that do not materially affect the basic and novel characteristic or characteristics of the claimed invention.

While the invention has been described in detail and with reference to specific embodiments thereof, it will be apparent to one skilled in the art that various changes and modifications can be made without departing from the scope of the invention.

## Claims

**1.** A method of preparing a stabilized ophthalmic composition, the method comprising:

(a) preparing an aqueous solution consisting essentially of: (1) ketotifen or a ketotifen salt in a concentration from 0.001% to 0.2% (w/v); (2) naphazoline or a naphazoline salt in a concentration from 0.001 % to 0.2% (w/v); (3) glycerol; (4) a preservative; and (5) water; and

(b) adjusting a pH value of the aqueous solution to an initial pH less than or equal to about 5;

thereby providing said stabilized ophthalmic composition, wherein the pH value of the ophthalmic composition remains between 4.3 to 4.8 when kept at 40 °C and 20% relative humidity for at least 10 days, wherein no more than 10% of the ketotifen or the ketotifen salt and no more than 5% of the naphazoline or the naphazoline salt are degraded when kept at 40°C and 20% relative humidity for at least 10 days, and wherein the composition is essentially free of buffering agents.

**2.** The method of claim 1, wherein said ketotifen or ketotifen salt is in a concentration from 0.01% to 0.05% (w/v), and said naphazoline or naphazoline salt is in a concentration from 0.01% to 0.1 % (w/v).

**3.** The method of claim 2, wherein said adjusting a pH comprises adding an adjusting agent that consists essentially of a solution of fumaric acid and sodium fumarate, or a solution of dilute hydrochloric acid.

**4.** The method of claim 2, wherein the glycerol is present at a concentration such that the solution has an osmolality of from 200 to 700 mOsm/kg.

**5.** The method of claim 2, wherein the glycerol is present at a concentration such that the solution has an osmolality of from 400 to 600 mOsm/kg.

**6.** The method of claim 2, wherein glycerol is present at a concentration from 1% to 6% (w/v).

**7.** A method of claim 1, wherein the preservative is benzalkonium chloride.

**8.** The method of claim 7, wherein said ketotifen or ketotifen salt is in a concentration from 0.01% to 0.05% (w/v); and said naphazoline or a naphazoline salt is in a concentration of from 0.01% to 0.1% (w/v).

**9.** The method of claim 1, wherein the ketotifen salt is ketotifen fumarate.

**10.** The method of claim 1, wherein the ketotifen salt is present in a concentration of from 0.01% to 0.05% (w/v).

**11.** The method of claim 1, wherein the naphazoline or naphazoline salt is present in a concentration of from 0.0 1 % to 0.1 % (w/v).

**12.** The method of claim 1, wherein the glycerol is present at a concentration such that the composition has an osmolality of from 200 to 700 mOsm/kg.

**13.** The method of claim 1, wherein the glycerol is present at a concentration such that the composition has an osmolality of from 400 to 600 mOsm/kg.

**14.** An ophthalmical composition comprising: (a) ketotifen or a ketotifen salt, which is present at a concentration from 0.001% to 0.2% (w/v); and (b) naphazoline or a naphazoline salt, which is present at a concentration from 0.001 % to 0.2% (w/v); wherein an initial pH of the composition is less than 5 and the pH remains at less than 5, when stored at 40 °C and 20% RH for at least 10 days and wherein no more than 10% of the ketotifen or the ketotifen salt and no more than 5% of the naphazoline or the naphazoline salt are degraded when kept at 40°C and 20% relative humidity for at least 10 days, and wherein the composition is essentially free of buffering agents.

**15.** The ophthalmical composition of claim 14, wherein the concentration of the ketotifen or a ketotifen salt is in a range from 0.001 % to 0.1 % (w/v).

**16.** The ophthalmical composition of claim 14, wherein the pH of the composition remains in a range from 4.3 to 5, when stored at 40 °C and 20% RH for at least 10 days.

**17.** The ophthalmical composition of claim 14, further comprising a pharmaceutically acceptable carrier.

**18.** The ophthalmical composition of claim 17, wherein the concentration of the ketotifen or a ketotifen salt and the concentration of the naphazoline or a naphazoline salt is in a range from 0.001% to 0.1% (w/v).

**Patentansprüche**

1. Ein Verfahren zur Herstellung einer stabilisierten ophthalmischen Zusammensetzung, wobei das Verfahren umfasst:

   (a) Herstellen einer wässrigen Lösung, im Wesentlichen bestehend aus: (1) Ketotifen oder einem Ketotifensalz in einer Konzentration von 0.001% bis 0.2% (w/V); (2) Naphazolin oder einem Naphazolinsalz in einer Konzentration von 0.001% bis 0.2% (w/V); (3) Glycerin; (4) einem Konservierungsmittel; und
   (b) Einstellen eines pH-Werts der wässrigen Lösung auf einen Ausgangswert kleiner oder gleich pH 5;

   wodurch besagte stabilisierte ophthalmische Zusammensetzung bereitgestellt wird, wobei der pH-Wert der ophthalmischen Zusammensetzung zwischen 4.3 und 4.8 bleibt, wenn die Zusammensetzung für mindestens 10 Tage bei 40 °C und 20% relativer Luftfeuchtigkeit gehalten wird, wobei nicht mehr als 10% des Ketotifens oder des Ketotifensalzes und nicht mehr als 5% des Naphazolins oder des Naphazolinsalzes zersetzt werden, wenn die Zusammensetzung für mindestens 10 Tage bei 40 °C und 20% relativer Luftfeuchtigkeit gehalten wird, und wobei die Zusammensetzung im Wesentlichen frei von Puffermitteln ist.

2. Das Verfahren gemäß Anspruch 1, wobei besagtes Ketotifen oder Ketotifensalz in einer Konzentration von 0.01% bis 0.05% (w/V), und besagtes Naphazolin oder Naphazolinsalz in einer Konzentration von 0.01% bis 0.1% (w/V) vorliegt.

3. Das Verfahren gemäß Anspruch 2, wobei das besagte Einstellen des pH-Werts die Zugabe eines Einstellmittels umfasst, das im Wesentlichen aus einer Fumarsäure- oder Natriumfumaratlösung, oder einer verdünnten Salzsäurelösung besteht.

4. Das Verfahren gemäß Anspruch 2, wobei Glycerin in einer Konzentration vorhanden ist, sodass die Lösung eine Osmolalität von 200 bis 700 mOsm/kg aufweist.

5. Das Verfahren gemäß Anspruch 2, wobei Glycerin in einer Konzentration vorhanden ist, sodass die Lösung eine Osmolalität von 400 bis 600 mOsm/kg aufweist.

6. Das Verfahren gemäß Anspruch 2, wobei Glycerin in einer Konzentration von 1% bis 6% (w/V) vorhanden ist.

7. Ein Verfahren gemäß Anspruch 1, wobei das Konservierungsmittel Benzalkoniumchlorid ist.

8. Das Verfahren gemäß Anspruch 7, wobei besagtes Ketotifen oder Ketotifensalz in einer Konzentration von 0.01% bis 0.05% (w/V), und besagtes Naphazolin oder Naphazolinsalz in einer Konzentration von 0.01% bis 0.1% (w/V) vorliegt.

9. Das Verfahren gemäß Anspruch 1, wobei das Ketotifensalz Ketotifenfumarat ist.

10. Das Verfahren gemäß Anspruch 1, wobei das Ketotifensalz in einer Konzentration von 0.01% bis 0.05% (w/V) vorhanden ist.

11. Das Verfahren gemäß Anspruch 1, wobei Naphazolin oder das Naphazolinsalz in einer Konzentration von 0.01 % bis 0.1 % (w/V) vorhanden ist.

12. Das Verfahren gemäß Anspruch 1 wobei Glycerin in einer Konzentration vorhanden ist, sodass die Lösung eine Osmolalität von 200 bis 700 mOsm/kg aufweist.

13. Das Verfahren gemäß Anspruch 1 wobei Glycerin in einer Konzentration vorhanden ist, sodass die Lösung eine Osmolalität von 400 bis 600 mOsm/kg aufweist.

14. Eine ophthalmische Zusammensetzung umfassend: (a) Ketotifen oder ein Ketotifensalz, das in einer Konzentration von 0.001% bis 0.2% (w/V) vorhanden ist; und (b) Naphazolin oder eine Naphazolinsalz, das in einer Konzentration von 0.001 % bis 0.2% (w/V) vorhanden ist; wobei der pH-Wert der Zusammensetzung einen Ausgangswert von kleiner als 5 aufweist und der pH-Wert kleiner 5 bleibt, wenn die Zusammensetzung für mindestens 10 Tage bei 40 °C und 20% relativer Luftfeuchtigkeit gelagert wird, und wobei nicht mehr als 10% des Ketotifens oder des Ketotifensalzes und nicht mehr als 5% des Naphazolins oder des Naphazolinsalzes zersetzt werden, wenn die Zusammensetzung für mindestens 10 Tage bei 40 °C und 20% relativer Luftfeuchtigkeit gehalten wird, und wobei die Zusammensetzung im Wesentlichen frei von Puffermitteln ist.

**15.** Die ophthalmische Zusammensetzung gemäß Anspruch 14, wobei die Konzentration an Ketotifen oder Ketotifensalz in einem Bereich von 0.001 % bis 0.1% (w/V) liegt.

**16.** Die ophthalmische Zusammensetzung gemäß Anspruch 14, wobei der pH-Wert der Zusammensetzung im Bereich von 4.3 bis 5 liegt, wenn die Zusammensetzung für mindestens 10 Tage bei 40 °C und 20% relativer Luftfeuchtigkeit gelagert wird.

**17.** Die ophthalmische Zusammensetzung gemäß Anspruch 14, weiter umfassend einen pharmazeutisch verträglichen Träger.

**18.** Die ophthalmische Zusammensetzung gemäß Anspruch 17, wobei die Konzentration an Ketotifen oder Ketotifensalz und die Konzentration an Naphazolin oder Naphazolinsalz in einem Bereich von 0.001% bis 0.1 % (w/V) liegt.

**Revendications**

**1.** Procédé de préparation d'une composition ophtalmique stabilisée, le procédé comprenant :

(a) la préparation d'une solution aqueuse essentiellement constituée : (1) de kétotifène ou d'un sel de kétotifène en une concentration de 0,001 % à 0,2 % (p/v) ; (2) de naphazoline ou d'un sel de naphazoline en une concentration de 0,001 % à 0,2 % (p/v) ; (3) de glycérol ; (4) d'un agent de conservation ; et (5) d'eau ; et
(b) l'ajustement de la valeur de pH de la solution aqueuse à un pH initial inférieur ou égal à environ 5 ;
pour ainsi produire ladite composition ophtalmique stabilisée, dans lequel la valeur de pH de la composition ophtalmique reste entre 4,3 et 4,8 lorsqu'elle est maintenue à une température de 40 °C et une humidité relative de 20 % pendant au moins 10 jours, dans lequel pas plus de 10 % du kétotifène ou du sel de kétotifène et pas plus de 5 % de la naphazoline ou du sel de naphazoline sont dégradés lorsqu'elle est maintenue à une température de 40 °C et une humidité relative de 20 % pendant au moins 10 jours, et dans lequel la composition est essentiellement exempte d'agents tampons.

**2.** Procédé selon la revendication 1, dans lequel ledit kétotifène ou ledit sel de kétotifène est présent en une concentration de 0,01 % à 0,05 % (p/v), et ladite naphazoline ou ledit sel de naphazoline est présent en une concentration de 0,01 % à 0,1 % (p/v).

**3.** Procédé selon la revendication 2, dans lequel ledit ajustement du pH comprend l'addition d'un agent d'ajustement qui est essentiellement constitué d'une solution d'acide fumarique et de fumarate de sodium, ou d'une solution d'acide chlorhydrique dilué.

**4.** Procédé selon la revendication 2, dans lequel le glycérol est présent en une concentration telle que la solution a une osmolalité de 200 à 700 mOsm/kg.

**5.** Procédé selon la revendication 2, dans lequel le glycérol est présent en une concentration telle que la solution a une osmolalité de 400 à 600 mOsm/kg.

**6.** Procédé selon la revendication 2, dans lequel le glycérol est présent en une concentration de 1 % à 6 % (p/v).

**7.** Procédé selon la revendication 1, dans lequel l'agent de conservation est du chlorure de benzalkonium.

**8.** Procédé selon la revendication 7, dans lequel ledit kétotifène ou ledit sel de kétotifène est présent en une concentration de 0,01 % à 0,05 % (p/v) ; et ladite naphazoline ou un sel de naphazoline est présent en une concentration de 0,01 % à 0,1 % (p/v).

**9.** Procédé selon la revendication 1, dans lequel le sel de kétotifène est du fumarate de kétotifène.

**10.** Procédé selon la revendication 1, dans lequel le sel de kétotifène est présent en une concentration de 0,01 % à 0,05 % (p/v).

**11.** Procédé selon la revendication 1, dans lequel la naphazoline ou le sel de naphazoline est présent en une concentration de 0,01 % à 0,1 % (p/v).

**12.** Procédé selon la revendication 1, dans lequel le glycérol est présent en une concentration telle que la composition a une osmolalité de 200 à 700 mOsm/kg.

**13.** Procédé selon la revendication 1, dans lequel le glycérol est présent en une concentration telle que la composition a une osmolalité de 400 à 600 mOsm/kg.

**14.** Composition ophtalmique comprenant : (a) du kétotifène ou un sel de kétotifène, qui est présent en une concentration de 0,001 % à 0,2 % (p/v) ; et (b) de la naphazoline ou un sel de naphazoline, qui est présent en une concentration de 0,001 % à 0,2 % (p/v) ; dans laquelle le pH initial de la composition est inférieur à 5 et le pH reste inférieur à 5, lorsqu'elle est stockée à une température de 40 °C et une HR de 20 % pendant au moins 10 jours et dans laquelle pas plus de 10 % du kétotifène ou du sel de kétotifène et pas plus de 5 % de la naphazoline ou du sel de naphazoline sont dégradés lorsqu'elle est maintenue à une température de 40 °C et une humidité relative de 20 % pendant au moins 10 jours, et dans laquelle la composition est essentiellement exempte d'agents tampons.

**15.** Composition ophtalmique selon la revendication 14, dans laquelle la concentration du kétotifène ou d'un sel de kétotifène se situe dans une plage de 0,001 % à 0,1 % (p/v).

**16.** Composition ophtalmique selon la revendication 14, dans laquelle le pH de la composition reste dans une plage de 4,3 à 5, lorsqu'elle est stockée à une température de 40 °C et une HR de 20 % pendant au moins 10 jours.

**17.** Composition ophtalmique selon la revendication 14, comprenant en outre un vecteur pharmaceutiquement acceptable.

**18.** Composition ophtalmique selon la revendication 17, dans laquelle la concentration du kétotifène ou d'un sel de kétotifène et la concentration de la naphazoline ou d'un sel de naphazoline se situe dans une plage de 0,001 % à 0,1 % (p/v).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6455547 B
- US 6576649 B
- WO 2006047418 A
- US 6274626 B
- US 20060148899 A
- US 2798053 A
- US 4192827 A
- US 4136250 A
- US 4548990 A
- US 6776982 B
- US 6468548 B
- US 20030031718 A
- WO 02100437 A